# EUROPEAN PATENT APPLICATION

(11) **EP 2 574 661 A1**
(43) Date of publication of application: **03.04.2013**
(21) Application number: 12184755.2
(22) Date of filing: 08.01.2008
(51) Int. Cl.: C12M 1/36, C12M 3/00, C12Q 1/68, B82Y 30/00

(54) **Nanoparticles useful for biomolecule storage**

(30) Priority: 16.01.2007 US 885206 P; 07.09.2007 US 970885 P
(62) Divisional of application: 08714205.5
(71) Applicant: GENVAULT CORPORATION, Carlsbad, CA 92011 (US)
(72) Inventor: Hogan, Michael, Carlsbad, CA 92011 (US)
(74) Representative: Vossius & Partner

(57) **Abstract**

The invention provides a composition useful for biomolecule storage comprising a population of nanoparticles. It also provides methods of storing a biomolecule and containers comprising the composition.

SEQUENCE LISTING

<110>  Genvault Corporation

<120>  NANOPARTICLES USEFUL FOR BIOMOLECULE STORAGE

<130>  P1561 EP/1

<150>  US 60/970,885
<151>  2007-09-07

<150>  US 60/885,206
<151>  2007-01-16

<160>  2

<170>  PatentIn version 3.5

<210>  1
<211>  27
<212>  DNA
<213>  Homo sapiens

<400>  1
agatgaagaa tgtgtgtgat ggatgta                                          27


<210>  2
<211>  24
<212>  DNA
<213>  Homo sapiens

<400>  2
gggctcgtaa ccataggaag ggta                                             24

## Description

### RELATED APPLICATIONS

This application claims priority of U. S. Provisional Application No. 60/885,206 filed on January 16, 2007, entitled: Nanoparticle-Based Storage of Biomolecules in the Dry State; and U.S. Provisional Application No. 60/970,885 filed on September 7, 2007, entitled: Nanoparticles Useful for Biomolecule Storage.

### BACKGROUND OF THE INVENTION

In many applications such as pharmaceutical and medical research, law enforcement, and military identification, for example, it is often desirable to store and to have access to numerous biological samples. Conventional biorepositories or other sample storage facilities utilize liquid or low temperature cryogenic systems for sample storage. These liquid and cryogenic systems are expensive both to create and to maintain. Additionally, current technology generally presents system operators with complicated and labor intensive maintenance and administrative responsibilities.

There is a need in the field to develop additional biomolecule storage materials and systems.

### SUMMARY OF THE INVENTION:

The present invention is based, in part, on the discovery that certain materials, especially nanoparticles are useful for the stabilization and/or storage of biomolecules. Accordingly, the present invention provides compositions, devices and methods useful for storing biomolecules.

In one embodiment of the invention, it provides a composition useful for biomolecule storage. The composition comprises a population of nanoparticles. The surface of the nanoparticles of the composition are passivated so that they are inert to a biomolecule.

In another embodiment of the invention, it provides a method of storing a biomolecule. The method comprises mixing a composition comprising a biomolecule with a population of nanoparticles of the invention to form a mixture, which is then dried for storage.

In yet another embodiment of the invention, it provides a multi-compartment container that comprises a dry storage matrix in a compartment of the container. The dry storage mix comprises a composition of a population of nanoparticles of the invention.

### BRIEF DESCRIPTION OF THE FIGURES

Figure 1 is a schematic view of a nanoparticle storage matrix.

Figure 2 is a schematic view of DNA storage clefts created from interstices among nanoparticles.

Figure 3 is a gel of PCR products from DNA recovered from dry state storage for 1 day and 3 days using a nanoparticle matrix composed of tungsten oxide passivated with borax. (See Example 11)

Figure 4 is a gel of PCR products from DNA recovered from dry state storage for 1 day and 3 days using a nanoparticle matrix composed of zirconium oxide passivated with borax. (See Example 12)

Figure 5 is a gel of PCR products from DNA recovered from dry-state storage for 10 and 34 days using nanoparticles composed of zirconium oxide or nanoparticles composed of tungsten oxide. (See Example 13)

Figure 6 is a gel of PCR products from DNA recovered after 7 days of dry storage using nanoparticles composed of zirconium oxide passivated with borax, and with the addition of glycerol as a plasticizer. (See Example 14)

Figure 7 is a gel of PCR products from DNA recovered after 10 days of dry state storage using a nanoparticle matrix composed of zirconium oxide passivated with borax. (See Example 15)

Figure 8 is a gel of PCR products from DNA recovered after 25 days of dry state storage using a nanoparticle matrix composed of zirconium oxide passivated with borax. (See Example 16)

### DETAILED DESCRIPTION OF THE INVENTION

As used herein, the following terms shall have the following meanings.

The terms "biopolymers" and "biomolecules" are used interchangeably herein and are expressly intended to include both short and long biopolymers including, but not limited to, such polymeric molecules as DNA, RNA, proteins, immunoglobulins, or carbohydrates, naturally existing or synthesized and with or without modified molecules, *e.g.*, modified amino acids or nucleotides. Thus, for example, the term includes both short (oligomeric) and long nucleic acid molecules, and similarly encompasses both short protein sequences (peptides) as well as longer polypeptides.

The term "protein" as used herein is used interchangeably with the term "polypeptide."

The term "nucleic acid," "oligonucleotide" and "polynucleotide" are used interchangeably and encompass DNA, RNA, cDNA, single stranded or double stranded and chemical modifications thereof.

The term "ceramic" is defined as an inorganic crystalline molecular solid with non-metallic properties comprising of elements from Group I, Group II, Group III Transition Metals, Group IV, Group V, Group VI, or Group VII of the periodic table, or mixtures including such elements.

The present invention is based, in part, on the discovery that certain materials, especially nanoparticles are useful for the stabilization and/or storage of biomolecules. Accordingly, the present invention provides compositions, devices and methods useful for storing biomolecules.

According to one aspect of the present invention, it provides a composition useful for biomolecule storage and such composition comprises a population of nanoparticles. Generally any nanoparticle, especially nanoparticles inert to one or more types of biomolecules can be used for the composition of the present invention. In one embodiment, the nanoparticles of the present invention are nanoparticles that have minimum or non-substantial interactions, *e.g.,* no direct binding interactions (covalent or non-covalent) or other chemical or physical interactions with one or more types of biomolecules.

In another embodiment, the surface of the nanoparticles of the present invention is modified or passivated so that the nanoparticles are inert or substantially non-interactive, non-reactive, or non-responsive to one or more types of biomolecules. For example, the surface of the nanoparticles can be treated or passivated so that it does not substantially retain biomolecules, *e.g.,* biomolecules can be retrieved from a liquid suspension of the nanoparticles of the present invention without significant adsorptive loss on the nanoparticle surface.

Exemplary means for modifying or passivating the surface of the nanoparticles of the present invention includes, without any limitation, passivating the surface with one or more passivating agents such as fluoride or an oxyanion, *e.g.*, borate, phosphate, sulfate, citrate, etc to weaken or minimize interactions between the nanoparticles of the present invention and biomolecules.

In an exemplary embodiment, nanoparticles made of ZrO₂, a substance that ordinarily has a very high affinity for nucleic acids is passivated for the storage of biomolecules. As illustrated in the examples herein, when passivated by an oxyanion, such as borate, ZrO₂ nanoparticles loose their affinity for nucleic acids and can be used directly as a dry state storage matrix for DNA. Other metal oxides, such as tungsten oxide, are also known to bind DNA and RNA and, when similarly passivated with an oxyanion, such as borate, these nanoparticle materials can be used as a nanoparticle matrix for the dry state storage of DNA and other biomolecules.

In yet another embodiment, the nanoparticles of the present invention are nanoparticles that are not necessarily substantially inert by themselves, however become inert in the presence of one or more suitable inactivating agents, optionally in an effective amount. In general, such inactivating agent can be any agent capable of binding to the surface of the nanoparticles of the present invention or excluding biomolecules from interacting with or binding to the surface of the nanoparticles of the present invention, *e.g.*, interfere or compete with biomolecules with respect to interacting with or binding to the surface of the nanoparticles of the present invention.

For example, inactivating agents can be agents that emulate the physical chemistry of the nucleic acids or other biomolecules, thus are capable of competing with biomolecules for the same surface binding sites. Alternatively inactivating agents can be agents capable of altering the binding or interacting environment for biomolecules with respect to nanoparticle surfaces, thus reducing or diminishing interaction between biomolecules and nanoparticle surfaces either in solution or solid phase or both. In addition, inactivating agents can be any suitable agents that present in an effective amount, *e.g.*, in substantial excess amount with respect to the surface of nanoparticles or the amount of biomolecules or both so that their presence interferes with biomolecules interaction with the surface of the nanoparticles of the present invention.

Exemplary inactivating agents include without any limitation certain nucleic acid competitors, *e.g.,* anionic proteins like albumin or casein, polymers known to be "hybridization blocking agents" such as polyvinyl pyrrolidone or dextran sulfate, anionic detergents such as SDS, or the corresponding lithium salt (LDS), neutral detergents such as Tween-20 or NP-40, zwitterionic detergents such as sarcosyl, small inorganic oxyanions such as borate, phosphate, vanidate, sulfate, halide anions such as fluoride (if the surface of the nanoparticles is ceramic), small organic oxyanions such as citrate, EDTA, EGTA, amine-sulfates of the "Goode" buffer series such as CAPS, CABS, MOPS, etc.

Exemplary inactivating agents also include without any limitation certain solution state "chaotropes" such as guandinium chloride, isothiocyanate, urea or formamide, which are capable of weakening DNA or RNA bond formation to the surface of the nanoparticles of the present invention or solid-state amendments capable of sequestering the nucleic acid in the dry state, such as borax, borax-glycerol, or cationic detergents like CTAB, that occlude nucleic acid binding to the surface of the nanoparticles of the present invention.

In addition, exemplary inactivating agents include certain biomolecule competitors capable of functioning as competitors when present in an effective amount, *e.g.*, in substantial access. Such inactivating agents include without any limitation the proteinacious complement of a crude chemical lysate of cells or tissues, the proteinacious complement of a crude protease digest of cells or tissue, any proteins or protein mixtures, any protein hydrozylates, water soluble polysaccharides such as dextran or ficol, poly-alcohols such as polyvinyl alcohol, and disaccharides such as sucrose, trehalose, maltose, etc.

According to the present invention, one or more inactivating agents can be present either in the composition containing the nanoparticles of the present invention or in the sample solution containing the biomolecules to be stored or a combination thereof. In one embodiment, one or more inactivating agents are present in unpurified or crude samples of biomolecules to be stored with the nanoparticles of the present invention. In another embodiment, one or more inactivating agents are present in an effective amount in crude samples containing nucleic acids.

According to the present invention, the nanoparticles of the present invention can be of any size suitable for storing biomolecules. In one embodiment, the nanoparticles have a diameter from about 10 nm to about 1000 nm, about 10 nm to about 500 nm, or from about 10 nm to about 400 nm. In another embodiment, the nanoparticles have a diameter from about 20 nm to about 400 nm, about 20 nm to about 300 nm, or about 20 nm to about 200 nm. In yet another embodiment, the nanoparticles have a diameter from about 20 nm to about 100 nm, or about 20 nm to about 30 nm. In still another embodiment, the nanoparticles have an average diameter of about 20 nm, about 25 nm, about 30 nm, about 35 nm, about 40 nm, about 45 nm or about 50 nm.

The nanoparticles of the present invention can be of any shape suitable for storing biomolecules. In one embodiment, the nanoparticles are spherical or nearly spherical in shape. In another embodiment, the nanoparticles are non-spherical, *e.g.*, they are cubes, ellipticals, posts, irregular shapes, etc.

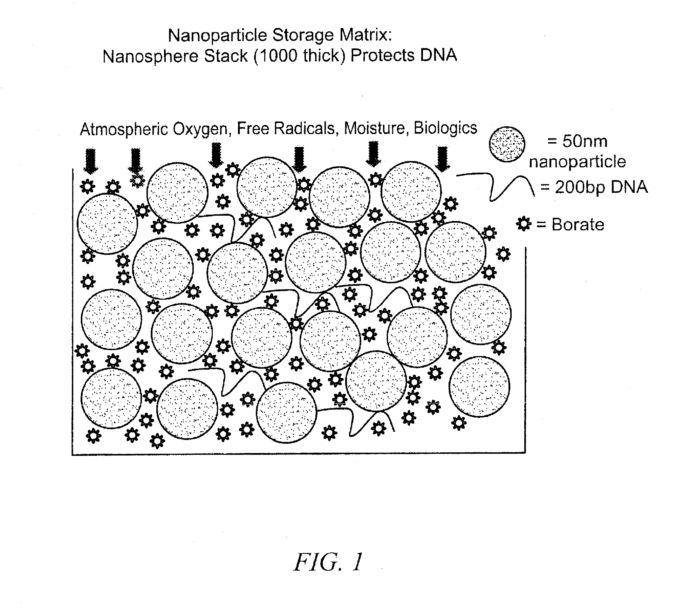

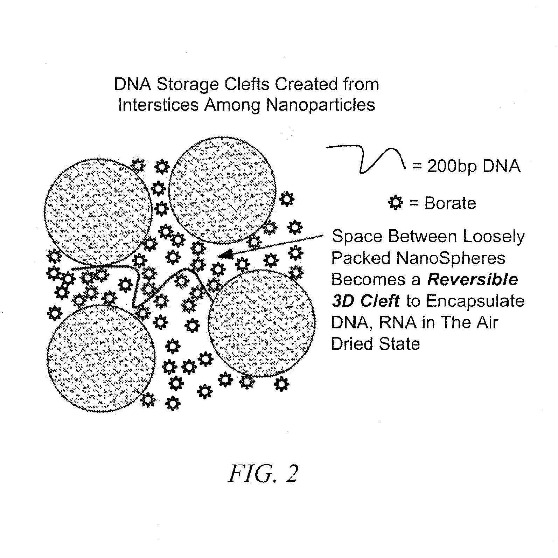

In general, the nanoparticles of the present invention, regardless of their shapes and sizes, when closely packed provide from about 15% to about 45%, from about 20% to about 40%, from about 25% to about 35% of unoccupied interstitial void volume, which is useful for storing biomolecules. For example, when spherical or nearly spherical non-porous particles are allowed to form a closely packed phase, a fraction of the total phase volume remains unoccupied by particles. For the orderly face-centered packing of spheres (like oranges in a grocery shelf), Gauss first calculated the unoccupied volume to be 26% (Conway and Sloane, 1993, Sphere Packings, Lattices, and Groups, 2nd ed. New York: Springer-Verlag). Torquato has calculated the unoccupied space for randomly packed spheres to be 34% (Torquato, et al., 2000, Is Random Close Packing of Spheres Well Defined?, Phys. Lev. Lett. 84:2064-2067). Also, Torquato has shown that randomly packed oblate ellipsoids (like M&Ms) pack a little better, with an unoccupied space of about 27% (Donev, et al., 2004, Improving the Density of Jammed Disordered Packings Using Ellipsoids, Science 303:990). (For exemplary illustrations see **Figure 1** and **Figure 2****).**

According to the present invention, the size of the interstitial space formed between the nanoparticles of the present invention is related to the size of the nanoparticles. For example, usually in a matrix formed by closely packed spheres, the cross-sectional diameter of the interstitial spaces formed between nearly spherical particles is approximately equivalent to the radius of the surrounding particles.

Specifically if closely packed nanoparticles of about 50 nm in diameter are used to form the biomolecule storage matrix, the unoccupied volume between the nanoparticles in the dried phase can be characterized by a series of connected chambers with an average cross-sectional diameter in the 25 nm range, also the radius of the nanoparticles. A chamber of average cross-sectional diameter in the 25 nm range is of a size which is generally larger than the width of a DNA, RNA, or protein molecule, but much smaller than the size of a bacterium or mold spore. Thus, in a dried state formed by closest packing of nanoparticles that are, *e.g.*, 50 nm spheres, large biomolecules can be sequestered in the interstitial space between spheres, but biologically active agents, such as bacteria or mold, would be excluded and could not contaminate the matrix. Thus in one embodiment, the nanoparticles of the present invention provide a convenient way of storing useful biomolecules and, at the same time, keeping out harmful contaminants or biohazardous agents such as bacteria. In an exemplary embodiment, 125 µg of the nanoparticles of the present invention comprising, *e.g.*, ceramic spheres in the fluid-free state form a dry phase of approximately 10 mm² in cross section and can be about 1,000 spheres thick.

The nanoparticles of the present invention can be made of a variety of substances. For example, the nanoparticles of the present invention can comprise metallic, semi-metallic, and/or non-metallic materials, including without any limitation, ceramics, clays, carbon-backboned or composite nanoparticles.

In one embodiment, the nanoparticles of the present invention comprise ceramic material. The ceramic material can be made of metal oxide or aluminum silicate. Examples of ceramic material include, but are not limited to, tungsten oxide, zirconium oxide, aluminum oxide, titanium oxide, tin oxide, phylosilicate, smectite, kaolin, bentonite, and combinations thereof. In another embodiment, the nanoparticles comprise branched polymers of a sugar, dextran or cellulose. Examples of a branched polymer of a sugar include, but are not limited to, polysucrose polymer, such as Ficoli. In yet another embodiment, the nanoparticles are non-porous.

According to the present invention, the nanoparticles of the present invention can be combined with one or more other agents to form compositions useful for biomolecule storage. Such agents can be any suitable entity that facilitates or enhances biomolecule storage using the nanoparticles of the present invention. Alternatively such agents can be any suitable entity that provides a function or characteristic useful for biomolecule storage using the nanoparticles of the present invention. In one embodiment, such agents include any biomolecule stabilizer, *e.g.*, small molecule stabilizer. In another embodiment, such agents include any small molecule additive. In yet another embodiment, such agents are any agents capable of inhibiting undesirable contact of biomolecules with various contaminants or potential sources of degradation, *e.g.*, oxygen, free water, enzymes, or other reactive chemical species.

In general, any agent capable of stabilizing biomolecules in the presence of the nanoparticles of the present invention is a biomolecule stabilizer. In one embodiment, the biomolecule stabilizer of the present invention is an agent capable of co-localization with biomolecules within interstitial spaces between nanoparticles in a dry state. For example, a biomolecule stabilizer includes any agent when added as part of a nanoparticle suspension, can concentrate, upon drying, into the interstitial spaces between nanoparticles to form a paracrystalline state in direct contact with the biomolecule. The co-localization of the biomolecule stabilizers and the biomolecules can provide additional stabilization of the biomolecule.

In another embodiment, the biomolecule stabilizer of the present invention is an agent capable of inhibiting microbial growth, *e.g.*, inhibiting bacterial, virus, or fungal growth. In yet another embodiment, the biomolecule stabilizer of the present invention is an agent capable of absorbing or sequestering water molecules, thereby preventing hydrolysis of the biomolecules. In still another embodiment, the biomolecule stabilizer of the present invention is capable of serving as a metal chelating agent so as to inhibit metal-dependant reactions to biomolecules. In yet still another embodiment, the biomolecule stabilizer of the present invention is a small molecule stabilizer. Examples of a biomolecule stabilizer include, but are not limited to, boric acid, sodium borate, boric acid-glycerol, boric acid-1,3 propanediol, sodium phosphate, CAPS, Na₂CO₃, EDTA, sodium lauroyl sarcosyl, guanidinium hydrochloride, SDS, urea, Tris, and combinations thereof.

According to the present invention, a small molecule additive can be any agent capable of facilitating or improving the nanoparticle storage function. In one embodiment, the small molecule additive of the present invention is an agent capable of improving the mechanical properties of the nanoparticle storage function. In another embodiment, the small molecule additive of the present invention is an agent capable of improving the durability of the nanoparticle storage function. In yet another embodiment, the small molecule additive of the present invention is an agent capable of facilitating the reversible dissociation between nanoparticles and biomolecules upon re-hydration of the composition. In still another embodiment, the small molecule additive of the present invention is an agent capable of facilitating or improving the nanoparticle storage function, but does not substantially interfere with the property of biomolecules, *e.g.*, does not interfere with the chemical, physical, or stability of biomolecules. In still yet another embodiment, the small molecule additive of the present invention is an agent capable of facilitating or improving the nanoparticle storage function as well as resisting microbial growth during storage.

One example of a small molecule additive is a plasticizer that can be used to improve the durability of the nanoparticle matrix and to facilitate the process of dissociation of the matrix once it is rehydrated. Other examples of a small molecule additive include, but are not limited to, polyols, *e.g.,* glycerol; simple monosaccharides; disaccharides; complex sugars; and dextrans. In one exemplary embodiment, the small molecule additive is glycerol. As described in the examples herein, glycerol, when added to sodium borate, improves the mechanical properties and the manufacturability of the dried nanoparticle storage matrix, *e.g.*, dried ZrO₂ nanoparticle storage matrix. For instance, addition of glycerol and sodium borate renders the dried nanoparticle composition (matrix) more resistant to vibrational damage, and facilitates reversible dissociation of the air-dried storage matrix upon re-hydration.

According to the present invention, the amount of other agents used in combination with the nanoparticles of the present invention can be any amount suitable for biomolecule storage. In one embodiment, the ratio of biomolecule stabilizer and small molecule additive is from about 1:5, 1:4, 1:3, 1:2, 1:1.5, 1:1, 1:0.5, or 1:0.1 by mass. In another embodiment, the ratio of nanoparticle and other agent, *e.g.*, biomolecule stabilizer and/or small molecule additive is from about 1:1 to about 20:1, from about 1.5:1 to about 15:1, from about 2:1 to about 10:1. In yet another embodiment, the mass ratio of nanoparticle to biomolecule stabilizer or small molecule additive or both can be from about 1:0.1 to about 1:10, from about 1:0.2 to about 1:5 or from about 1:0.5 to about 1:2.

In one exemplary embodiment, the biomolecule stabilizer is borate while the small molecule additive is glycerol and the molecular ratio of glycerol to borate is from about 0.5 glycerol to 1 borate ion up to about 2 glycerol to 1 borate ion; about 1 glycerol to 1 borate ion, or about 1.5 glycerol to 1 borate ion. In another exemplary embodiment, the nanoparticles are ZrO₂ particles and the mass ratio of the nanoparticles, *e.g.*, of ZrO₂ particles, to the borate ion-glycerol mix is about 1:1, about 2:1, about 3:1, about 5:1, about 7:1, or about 10:1 by mass.

The nanoparticles of the present invention, optionally including one or more other agents can be provided as a nanoparticle composition in any form suitable for biomolecule storage. In one embodiment, the nanoparticle compositions of the present invention are provided in a suspension, *e.g.*, colloidal suspension, aqueous, or wet form. In another embodiment, the nanoparticle compositions of the present invention are provided in a solid or dry form, *e.g.*, air-dried form, which can be converted to a liquid form, *e.g.,* by suspending or re-hydrate the composition with a suitable solution, *e.g.*, water, buffer, etc.

In yet another embodiment, the nanoparticle compositions of the present invention are provided in a solid or dry form or any other pre-finishing stage and with an instruction for converting it to a form suitable for biomolecule storage, *e.g.*, converting it to a liquid form so that it can be mixed with biomolecules and subsequently dried for storage.

In still another embodiment, the nanoparticle compositions of the present invention are provided in a multi-compartment container, *e.g.*, in a wet or dry form in a multi-well plate. For example, each compartment contains the nanoparticle composition of the present invention, which is ready to receive a biomolecule sample for storage. In general, such multi-compartment container is compatible with automation and robotic handling.

According to the present invention, the nanoparticle composition of the present invention can be mixed with biomolecules in any manner suitable for storage. In general, a biomolecule sample can be added to the nanoparticle composition of the present invention, *e.g.*, a suspension of the nanoparticle composition to form a mixture, which subsequently can be dried in any suitable way, *e.g.*, air-dry to form a fluid-free air-dried storage matrix comprising the nanoparticles and biomolecules. In one embodiment, the nanoparticle composition of the present invention is mixed as a colloidal solution with biomolecules to be stored. It is generally believed (without being bound to any limitation) that nanoparticles can spontaneously assemble during the process of air-drying to form a matrix which approximates a three-dimensional volume of closely packed nanoparticles with spaces formed between the nanoparticles available to sequester biomolecules, *e.g.*, drying process results in a dried solid matrix where biomolecules are encapsulated within the interstitial spaces.

In one exemplary embodiment, the nanoparticles comprise a ceramic material and upon re-hydration of a solid matrix of the nanoparticles and biomolecules, the nanoparticles are dispersed as a colloidal suspension and the biomolecules are recovered from the matrix by, *e.g.*, centrifugation whereby the nanoparticles form a pellet and the biomolecules remain in solution. In another exemplary embodiment, the nanoparticles comprise a branched polymer of a sugar and upon re-hydration of a solid matrix of the nanoparticles and biomolecules, the matrix dissociates to form a dilute aqueous suspension, allowing the stored biomolecules to partition freely into the fluid phase, *e.g.*, free from diffusional impediment associated with the nanoparticles.

In general, the nanoparticle composition of the present invention can be used to store any biomolecule. Exemplary biomolecules include without any limitation, DNA, RNA, nucleic acid, polynucleotide, oligonucleotide, amino acid, peptide, polypeptide. Such biomolecules can be in any form, *e.g.*, in a biological sample, an extract, or any other intermediate or semi-processed biological samples. Exemplary biological samples include without any limitation, blood, plasma, urine, saliva, spinal fluid, or any biological fluid, skin cells, cell or tissue samples, cell lysate, nuclear extract, nucleic acid extract, protein extract, cytoplasmic extract, etc.

In one embodiment, the nanoparticle composition of the present invention includes nanoparticles of a ceramic or clay and the biomolecules to be stored are nucleic acids or samples comprising nucleic acids. In another embodiment, the nanoparticle composition of the present invention includes nanoparticles of branched polymers of sugar, dextran or cellulose and the biomolecules to be stored are peptides or polypeptides or samples comprising peptides or polypeptides.

### EXAMPLES

The following examples are intended to illustrate, but not to limit, the invention in any manner, shape, or form, either explicitly or implicitly. While they are typical of those that might be used, other procedures, methodologies, or techniques known to those skilled in the art may alternatively be used.

### Example 1: Borate-Passivated Zirconium Oxide as a Nanoparticle Storage Matrix

Zirconium oxide nanoparticles (having an average diameter of about 25 nm (and a full diameter range from about 20 nm to about 100 nm) were suspended at 10% by weight in 1M HCl and incubated at room temperature for about 16 hours with constant mixing to passivate the particles. The particles were then centrifuged at 5,000 G to form a pellet and then resuspended in 0.1 M NaCl. The washing process was repeated until the resulting supernatant had a pH of 5 or greater. The particles were then resuspended to 10% by weight in 100 mM borax (disodium tetraborate) and incubated for at least 16 hours. This suspension was subjected to sedimentation at 5000 G and then resuspended in 10 mM borax at a particle concentration of 6.25 mg/mL to be used as a stock solution.

### Example 2: Borate-Passivated Tungsten Oxide as a Nanoparticle Storage Matrix

The process was the same as that described in Example 1 with the exception that the nanoparticle was composed of tungsten oxide having an average diameter of 25 nm, and a full diameter range from about 20 nm to about 100 nm.

### Example 3: ZrO₂ Nanoparticle Storage Matrix in a Standard 96-well Round Bottom Polypropylene Microtiter Plate

A nanoparticle storage matrix was fabricated in a round bottom, polypropylene, 96 well microtiter plate. 20µL of total fluid was added per well composed of 125 µg of the ZrO₂ nanoparticles of Example 1 and 40 µg of borax. The added 20 µL suspension was air dried at room temperature onto the bottom surface of each well to form a discrete pellet of the storage matrix. The drying process typically required 16 hours of incubation.

### Example 4: WO₃ Nanoparticle Storage Matrix in a Standard 96-well Round Bottom Polypropylene Microtiter Plate

The process was the same as that described in Example 3 with the exception that the nanoparticle component is tungsten oxide as described in Example 2. The 20 µL suspension was air dried at room temperature onto the bottom surface of each well of a 96 well microtiter plate for at least 16 hours.

### Example 5: ZrO2 Nanoparticle Storage Matrix with Glycerol

The process is the same as described in Example 3 with the addition of glycerol at a total mass of 55 µg per 20 µL of total suspension. The mixed suspension was air dried at room temperature onto the bottom surface of each well for at least 16 hours.

### Example 6: ZrO₂ Nanoparticle Storage Matrix with Glycerol

The process was the same as described in Example 5 with the exception that glycerol was added to 110 µg per 20 µL of total suspension. The mixed suspension was air dried at room temperature onto the bottom surface of each well for at least 16 hours.

### Example 7: ZrO₂ Nanoparticle Storage Matrix with Glycerol

The process was the same as described in Example 5 with the exception that glycerol was added to 165 µg per 20 µL. The mixed suspension was air dried onto the bottom surface of each well for at least 16 hours at room temperature.

### Example 8: Storage of DNA in an Air Dried Nanoparticle Matrix in a Standard 96-well Round Bottom Polypropylene Microtiter Plate

To the air-dried nanoparticle pellets described in Example 3, Example 4, Example 5, Example 6, and Example 7, a solution of human DNA was added in TE buffer (Roche Gen) at a volume of 20 µL per well. The dried nanoparticle matrix of each well was resuspended in this DNA solution by pipetting to confluence. The resulting suspension was then air-dried back to a dried nanoparticle pellet in the same well. The amount of DNA added to each well ranged from about 1 ng to about 30 ng. After the air-dried nanoparticle matrix was formed with the DNA sample, the plate was stored at either room temperature, (approximately 25°C) or at 56°C for up to 24 days.

### Example 9: Recovery of DNA from the Air Dried Nanoparticle Matrix by Resuspension in Water

To retrieve the DNA from the air-dried nanoparticle matrix of Example 8, 20 µL of water was added to each well and incubated at room temperature for about 15 minutes. The matrix was dissociated by repeated pipetting until homogenous, The resulting nanoparticle suspension was then incubated at either 56°C or at room temperature for a minimum of 30 minutes, with occasional pipetting or vortex mixing to ensure a confluent nanoparticle suspension. After that incubation, the suspension was subjected to centrifugation at 8,000 G or greater for 3 minutes to pellet the spent nanoparticles. The DNA-containing supernatant above the spent nanoparticle pellet was retrieved by pipetting and then used "as is" or diluted for further analysis.

### Example 10: PCR Analysis of Human DNA

PCR was used to compare and evaluate the DNA recovery process from the nanoparticles of the invention as described in the following Examples. These PCR analyses were based on a nuclear chromosome encoded gene, amelogenin, encoded on both the X and Y chromosomes.

The primers used were of two sequences. The sequence of the first primer was 5' - AGA TGA AGA ATG TGT GTG ATG GAT GTA -3' (SEQ ID NO:1), and the sequence of the second primer was 5' - GGG CTC GTA ACC ATA GGA AGG GTA - 3' (SEQ ID NO:2). Both sequences were derived from the amelogenin sequence in GenBank with accession number AY040206. The PCR product from these two primers is a 558 base pair long fragment. In general, PCR reactions were carried out as follows. The PCR reactions were carried out in a 50µL volume. The reactions contained 1 x Roche PCR Buffer, 1.5 mM MgCl₂, 0.4 µM primers, 0.2 mM dNTPs, 0.16 mg/ml BSA, and 0.4µL of Fast Start Taq at 5 U/µL. The conditions for these PCR tests were as follows. The first step was at 94°C for 4 minutes. Then there were 35 cycles composed of three steps including 94°C for 1 minute, followed by 65°C for I minute, and then 72°C for 1 minute. After these 35 cycles, the reactions were incubated at 72°C for 7 minutes followed by a holding step at 15°C until the reactions were stopped. All of PCR results were evaluated by electrophoresis of 1/5 of the reaction volume in agarose gels using a Tris-Borate-EDTA buffer system. The molecular weight control used was the 1 Kb DNA ladder from Invitrogen (catalog no. 15615-016). The PCR controls used were a negative control, a reaction with no added DNA template, and four positive controls with a fixed and known amount of human DNA (Roche Human Genomic DNA catalog no. 1691112) used as PCR templates, generally at concentrations of 10 ng, 1 ng, 0.1 ng and 0.01 ng per 50 µL PCR reaction.

### Example 11: PCR Analysis of DNA Recovered from Dry State Storage for 1 Day and 3 Days, Nanoparticle Matrix Composed of WO₃ Passivated with Borax

This example is a PCR analysis of DNA recovered from dry state storage for 1 day and 3 days. The nanoparticle matrix was composed of WO₃ passivated with borax as described in Example 4. PCR assays were done in a 25µL volume, using 4µL, of DNA as template for all reactions. All template DNA samples were adjusted to a dilution of 0.05 ng per microliter (i.e. 0.2 ng per reaction), assuming 100% recovery of the original input DNA. The PCR target sequence was the human amelogenin locus which yields a 558 bp amplicon and is described in detail above in Example 10. The PCR product was analyzed by 2% agarose electrophoresis with Tris-borate buffer at 150 volts for 45 minutes.

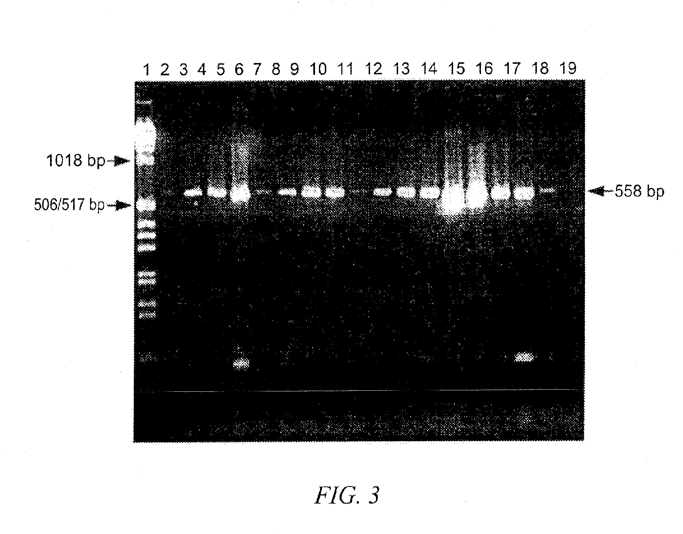

The results are shown in **Figure 3** and demonstrate the reversible DNA recovery from a WO₃ nanoparticle matrix passivated with borax for up to three days of room temperature dry state storage. Referring to **Figure 3****,** lane 1 contains a double-stranded DNA molecular weight marker. Lanes 2-5 contain DNA samples extracted after one hour as a nanoparticle slurry. Lanes 6-9 contain a DNA samples extracted after one day of dry-state storage within a WO₃/borax nanoparticle matrix. Lanes 10-13 contain a DNA samples extracted after three days dry-state storage within a WO₃/borax nanoparticle matrix. Lanes 14-18 contain semi-quantitative-PCR controls using various known amounts of human DNA, and lane 19 is the negative PCR control, with no DNA in the assay.

More specifically, in **Figure 3**, Lane 2 contains a DNA sample of 1 ng extracted after one hour as a nanoparticle slurry. Lane 3 contains a DNA sample of 3 ng extracted after one hour as a nanoparticle slurry. Lane 4 contains a DNA sample of 10 ng extracted after one hour as a nanoparticle slurry. Lane 5 contains a DNA sample of 30 ng extracted after one hour as a nanoparticle slurry. Lane 6 contains a DNA sample of 1 ng extracted after one day of dry-state storage within a VO₃/borax nanoparticle matrix. Lane 7 contains a DNA sample of 3 ng extracted after one day dry-state storage within a WO₃/borax nanoparticle matrix. Lane 8 contains a DNA sample of 10 ng extracted after one day dry-state storage within a WO₃/borax nanoparticle matrix. Lane 9 contains a DNA sample of 30 ng extracted after one day dry-state storage within a WO₃/borax nanoparticle matrix. Lane 10 contains a DNA sample of 1 ng extracted after three days dry-state storage within a WO₃/bora nanoparticle matrix. Lane 11 contains a DNA sample of 3 ng extracted after three days dry-state storage within a WO₃/borax nanoparticle matrix. Lane 12 contains a DNA sample of 10 ng extracted after three days dry-state storage within a WO₃/borax nanoparticle matrix. Lane 13 contains a DNA sample of 30 ng extracted after three days dry-state storage within a WO₃/borax nanoparticle matrix. Lane 14 contains a semi-quantitative-PCR control of 10 ng of human DNA input per assay. Lane 15 is a semi-quantitative-PCR control of 1.0 ng of human DNA input per assay. Lane 16 is a semi-quantitative-PCR control of 0.2 ng of human DNA input per assay. Lane 17 is a semi-quantitative-PCR control of 0.10 ng of human DNA input per assay. Lane 18 is a semi-quantitative-PCR control of 0.001 ng of human DNA input per assay. Lane 19 is a negative PCR control in which no human DNA was added per assay.

### Example 12: PCR Analysis of DNA Recovered from Dry State Storage for 1 Day and 3 Days, Nanoparticle Matrix Composed of ZrO₂ Passivated with Borax

This example is a PCR analysis of DNA recovered from dry state storage for 1 day and 3 days using a nanoparticle matrix composed of ZrO₂ passivated with borax as described in Example 3. PCR assays were done in a 25µL volume, using 4µL of DNA as template for all reactions. All template DNA samples were adjusted to a dilution of 0.05 ng per microliter (i.e. 0.2 ng per reaction), assuming 100% recovery of the original input DNA. The PCR target sequence was the human amelogenin locus which yields a 558 bp amplicon and is described in detail above in Example 10. The PCR product was analyzed by 2% agarose electrophoresis with Tris-borate buffer at 150 volts for 45 minutes.

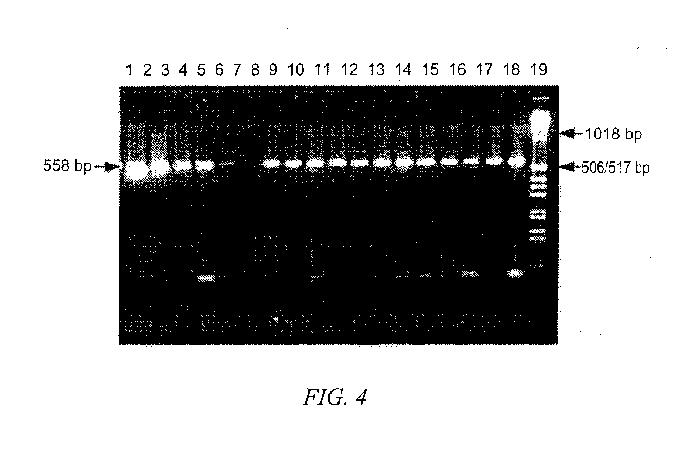

The results are shown in **Figure 4** and demonstrate the reversible DNA recovery from a ZrO₂ nanoparticle matrix passivated with borax for up to three days of room temperature dry state storage. The data also show that, at the formulations used, recovery with the ZrO₂ nanoparticle matrix was more efficient than recovery with the WO₃ nanoparticle matrix of Example 11. Referring to **Figure 4****,** lanes 1-5 are semi-quantitative PCR controls using known amounts of human DNA. Lane 6 is a negative PCR control in which no human DNA was added. Lanes 7-10 contain DNA samples extracted after one hour as a nanoparticle slurry. Lanes 11-14 contain DNA samples extracted after one day dry-state storage within a ZrO₂/borax nanoparticle matrix. Lanes 15-18 contain a DNA samples extracted after three days dry-state storage within a ZrO₂/borax nanoparticle matrix. Lane 19 is a double-stranded DNA molecular weight marker.

More specifically, in **Figure 4****,** lane 1 is a semi-quantitative PCR control using 10 ng of human DNA input per assay. Lane 2 is a semi-quantitative PCR control using 1.0 ng of human DNA input per assay. Lane 3 is a semi-quantitative PCR control using 0.2 ng of human DNA input per assay. Lane 4 is a semi-quantitative PCR control using 0.10 ng of human DNA input per assay. Lane 5 is a semi-quantitative PCR control using 0.001 ng of human DNA input per assay. Lane 6 is a negative PCR control in which no human DNA was added per assay. Lane 7 contains a DNA sample of 1 ng extracted after one hour as a nanoparticle slurry. Lane 8 contains a DNA sample of 3 ng extracted after one hour as a nanoparticle slurry. Lane 9 contains a DNA sample of 10 ng extracted after one hour as a nanoparticle slurry. Lane 10 contains a DNA sample of 30 ng extracted after one hour as a nanoparticle slurry. Lane 11 contains a DNA sample of I ng extracted after one day dry-state storage within a ZrO₂/borax nanoparticle matrix. Lane 12 contains a DNA sample of 3 ng extracted after one day dry-state storage within a ZrO₂/borax nanoparticle matrix. Lane 13 contains a DNA sample of 10 ng extracted after one day dry-state storage within a ZrO₂/borax nanoparticle matrix. Lane 14 contains a DNA sample of 30 ng extracted after one day dry-state storage within a ZrO₂/borax nanoparticle matrix. Lane 15 contains a DNA sample of 1 ng extracted after three days dry-state storage within a ZrO₂/borax nanoparticle matrix. Lane 16 contains a DNA sample of 3 ng extracted after three days dry-state storage within a ZrO₂/borax nanoparticle matrix. Lane 17 contains a DNA sample of 10 ng extracted after three days dry-state storage within a ZrO₂/borax nanoparticle matrix. Lane 18 contains a DNA sample of 30 ng extracted after three days dry-state storage within a ZrO₂/borax nanoparticle matrix. Lane 19 is a double-stranded DNA molecular weight marker.

### Example 13: Comparison of Dry-State Storage of DNA for 10 or 34 days, on Nanoparticles Composed of Zirconium Oxide or Tungsten Oxide

This example compares the dry-state storage of DNA for 10 and 34 days using either nanoparticles composed of zirconium oxide prepared according to Example 3 or nanoparticles composed of tungsten oxide prepared according to Example 4.

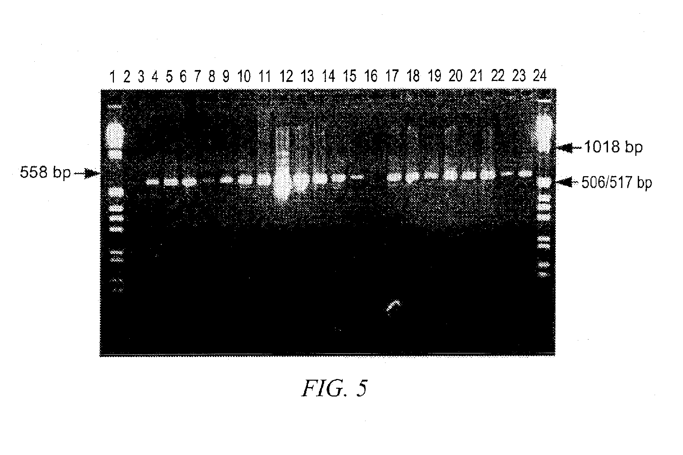

PCR assays were done in a 25µL volume, using 4µL of DNA as template for all reactions. All template DNA samples were adjusted to a dilution of 0.05 ng per microliter (i.e. 0.2 ng per reaction), assuming 100% recovery of the original input DNA. The PCR target sequence was the human amelogenin locus which yields a 558 bp amplicon and is described in detail above in Example 10. The PCR product was analyzed by 2% agarose electrophoresis with Tris-borate buffer at 150 volts for 45 minutes. As seen in **Figure 5**, data demonstrate that recovery with the ZrO₂ nanoparticle matrix remains more efficient than recovery with the WO₃ nanoparticle matrix after 10 or 34 days of dry state storage.

Referring to **Figure 5****,** lane 1 is a double-stranded DNA molecular weight marker. Lanes 2-5 contain DNA samples extracted after ten days dry-state storage within a WO₃ nanoparticle matrix. Lanes 6-9 contain DNA samples of 1 ng extracted after thirty four days dry-state storage within WO₃ nanoparticle matrix. Lanes 10-14 are semi-quantitative PCR controls using known quantities of human DNA. Lane 15 is a negative PCR control in which no human DNA was added. Lanes 16-19 contain DNA samples of 1 ng extracted after ten days dry-state storage within ZrO₂ nanoparticle matrix. Lanes 20-23 contain DNA samples extracted after thirty four days dry-state storage within ZrO₂ nanoparticle matrix.

More specifically, in **Figure 5****,** lane 1 is a double-stranded DNA molecular weight marker. Lane 2 contains a DNA sample of 1 ng extracted after ten days dry-state storage within a WO₃ nanoparticle matrix. Lane 3 contains a DNA sample of 3 ng extracted after ten days dry-state storage within WO₃ nanoparticle matrix. Lane 4 contains a DNA sample of 10 ng extracted after ten days dry-state storage within WO₃ nanoparticle matrix. Lane 5 contains a DNA sample of 30 ng extracted after ten days dry-state storage within WO₃ nanoparticle matrix. Lane 6 contains a DNA sample of 1 ng extracted after thirty four days dry-state storage within WO₃ nanoparticle matrix. Lane 7 contains a DNA sample of 3 ng extracted after thirty four days dry-state storage within WO₃ nanoparticle matrix. Lane 8 contains a DNA sample of 10 ng extracted after thirty four days dry-state storage within WO₃ nanoparticle matrix. Lane 9 contains a DNA sample of 30 ng extracted after thirty four days dry-state storage within WO₃ nanoparticle matrix. Lane 10 is a semi-quantitative PCR control of 10 ng of human DNA input per assay. Lane 11 is a semi-quantitative PCR control of 1.0 ng of human DNA input per assay. Lane 12 is a semi-quantitative PCR control of 0.2 ng of human DNA input per assay. Lane 13 is a semi-quantitative PCR control of 0.10 ng of human DNA input per assay. Lane 14 is a semi-quantitative PCR control of 0.001 ng of human DNA input per assay. Lane 15 is a negative PCR control in which no human DNA input was added per assay. Lane 16 contains a DNA sample of 1 ng extracted after ten days dry-state storage within ZrO₂ nanoparticle matrix. Lane 17 contains a DNA sample of 3 ng extracted after ten days dry-state storage within ZrO₂ nanoparticle matrix. Lane 18 contains a DNA sample of 10 ng extracted after ten days dry-state storage within ZrO₂ nanoparticle matrix. Lane 19 contains a DNA sample of 30 ng extracted after ten days dry-state storage within ZrO₂ nanoparticle matrix. Lane 20 contains a DNA sample of 1 ng extracted after thirty four days dry-state storage within ZrO₂ nanoparticle matrix. Lane 21 contains a DNA sample of 3 ng extracted after thirty four days dry-state storage within ZrO₂ nanoparticle matrix. Lane 22 contains a DNA sample of 10 ng extracted after thirty four days dry-state storage within ZrO₂ nanoparticle matrix. Lane 23 contains a DNA sample of 30 ng extracted after thirty fours day dry-state storage within ZrO₂ nanoparticle matrix.

### Example 14: PCR Analysis of DNA Recovered from 7 Days of Dry State Storage using a Nanoparticle Matrix Composed of ZrO₂ Passivated with Borax

This experiment analyzed DNA recovered from 7 days of dry storage using two different nanoparticle matrices, each composed of ZrO₂ passivated with borax, and each with the addition of glycerol as a plasticizer, as described in Examples 5 and 6. Storage conditions were tested with each type of nanoparticle at two storage temperatures, room temperature and 56°C.

In this experiment, different storage conditions were evaluated. All nanoparticle slurry samples were dried at room temperature for 16 hours. The two types of nanoparticles used were ZrO₂ nanoparticles with borax and with differing amounts of added glycerol. The nanoparticles of Example 5 were used for samples 1-8 and the nanoparticles of Example 6 were used for samples 9-16. Two different post drying storage conditions were also tested. Samples 5-8 and 13-16 were stored at room temperature, and samples 1-4 and samples 9-12 were stored at 56°C. Elution conditions were at room temperature for 60 minutes followed by 56°C for 30 minutes. All samples were eluted in 20µL of water. For each sample, the amount tested was adjusted to a DNA input of 0.2 ng, assuming 100% recovery of the DNA.

PCR assays were done in a 25µL volume, using 4µL of DNA as template for all reactions. All template DNA samples were adjusted to a dilution of 0.05 ng per microliter (i.e. 0.2 ng per reaction), assuming 100% recovery of the original input DNA. The PCR target sequence was the human amelogenin locus which yields a 558 bp amplicon as described in detail above in Example 10. The PCR product was analyzed by 2% agarose electrophoresis with Tris-borate buffer at 150 volts for 45 minutes.

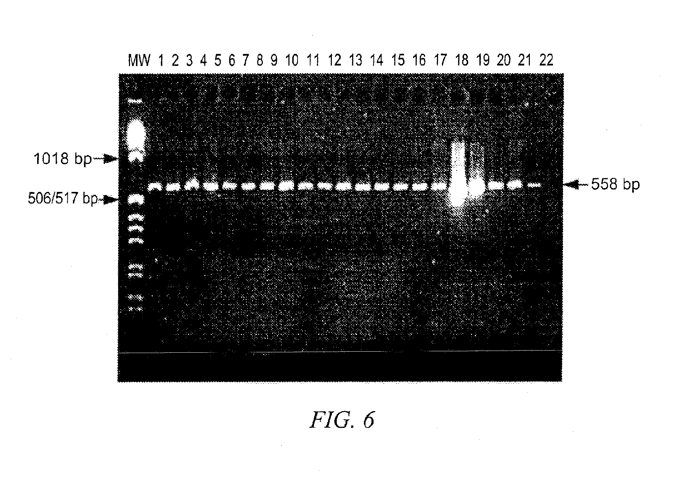

Referring to **Figure 6**, PCR assays of the 1 ng samples, in lanes 1, 5, 9, and 13, tested 4 µL of the 20µL eluate. PCR assays of the 3 ng samples, in lanes 2, 6, 10, and 14, tested 4 µL of the 1/3 dilution of the 20 µL eluate. PCR assay of the 10 ng samples, in lanes 3, 7,11. and 15, tested 4 µL of the 1/10 dilution of the 20 µL eluate. PCR assays of the 30 ng samples, in lanes 4, 8, 12, and 16 tested 4 µL of the 1/30 dilution of the 20 µL eluate. Lanes 17-21 are PCR controls containing known amounts of human DNA with 10 ng in lane 17, 1.0 ng in lane 18, 0.2 ng in lane 19, 0.1 ng in lane 20, and 0.01 ng in lane 21. The lane marked MW contains a double-stranded DNA molecular weight marker, and lane 22 contains a negative PCR control with no DNA.

As seen in **Figure 6**, these data demonstrate reversible DNA recovery from a ZrO₂ nanoparticle matrix passivated with borax, with the addition of glycerol as a plasticizer at two plasticizer amounts, out to 7 days of room temperature dry state storage. The data show that, with both of the nanoparticle formulations (Examples 5 and 6), recovery with the ZrO₂ nanoparticle matrix, with glycerol as an additive, approached 100%.

### Example 15: PCR Analysis of DNA Recovered from 10 Days of Dry State Storage. Nanoparticle Matrix Composed of ZrO₂ Passivated with Borax

The experiment is a PCR analysis of DNA recovered after 10 days of dry state storage using a nanoparticle matrix composed of ZrO₂ passivated with borax as described in Examples 5 and 6. PCR assays were done in a 25µL volume, using 4µL of DNA as template for all reactions. All template DNA samples were adjusted to a dilution of 0.05 ng per microliter (i.e. 0.2 ng per reaction), assuming 100% recovery of the original input DNA. The PCR target sequence was the human amelogenin locus which yields a 558 bp amplicon and is described in detail in Example 10. The PCR product was analyzed by 2% agarose electrophoresis with Tris-borate buffer at 150 volts for 45 minutes.

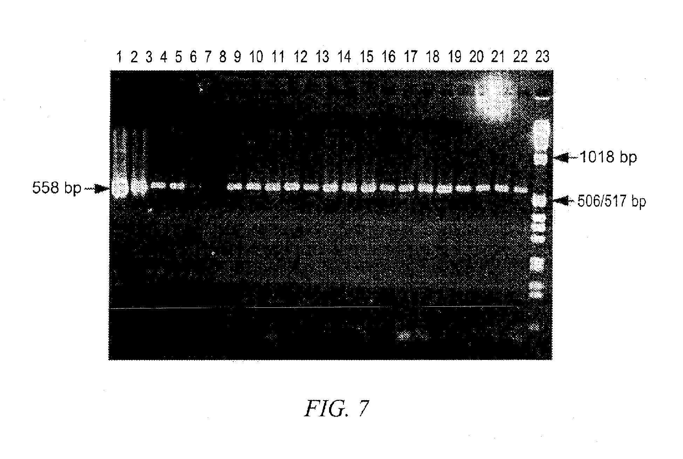

As seen in **Figure 7**, these data demonstrate reversible DNA recovery from a ZrO₂ nanoparticle matrix passivated with borax with the addition of glycerol as a plasticizer at two plasticizer amounts (as in Example 5 and Example 6) out to 10 days of room temperature dry state storage. The data also show that, for both of the nanoparticle formulations used, recovery with the ZrO₂ nanoparticle matrix, with glycerol as an additive, approached 100%. The data also show that the DNA the quality and the quantity of the recovered DNA, as a PCR template, do not appear to be affected by raising the storage temperature to 56°C.

Referring to **Figure 7**, lanes 1-5 are PCR controls containing known amounts of human DNA with 10 ng in lane 1, 1.0 ng in lane 2, 0.2 ng in lane 3, 0.1 ng in lane 4, and 0.01 ng in lane 5. Lane 6 is a negative PCR control with no DNA. Lanes 7-14 contain PCR products from DNA extracted after 10 days dry storage using the nanoparticles of Example 5. Lanes 7-10 show the products from DNA stored at room temperature, and lanes 11-14 show the products from DNA stored at 56°. Lanes 15-22 contain PCR products from DNA extracted after 10 days dry storage using the nanoparticles of Example 6. Lanes 15-18 show the products from DNA stored at room temperature, and lanes 19-22 show the products from DNA stored at 56°. Lanes 7, 9, 11, 13, 15, 17, 19, and 21 contain the products from DNA eluted at room temperature, and lanes 8, 10, 12. 14, 16, 18, 20, and 22 contain the products from DNA eluted at 56°. Additionally, lanes 7, 8, 11, 12, 15, 16, 19, and 20 are PCR assays using 1 ng of DNA, and lanes 9, 10, 13, 14, 17, 18, 21, and 22 are PCR assays using 3 ng of DNA. Lane 23 is a double-stranded DNA molecular weight marker.

### Example 16: PCR Analysis of DNA Recovered from 25 Days of Dry State Storage Using a Nanoparticle Matrix Composed of ZrO₂ Passivated with Borax

The experiment is a PCR analysis of DNA recovered from 25 days of dry state storage using a nanoparticle matrix composed of ZrO₂ passivated with borax as described in Example 5. PCR assays were done in a 25µL volume, using 4µL of DNA as template for all reactions. All template DNA samples were adjusted to a dilution of 0.05 ng per microliter (i.e. 0.2 ng per reaction), assuming 100% recovery of the original input DNA. The PCR target sequence was the human amelogenin locus which yields a 558 bp amplicon and is described in detail in Example 10. The PCR product was analyzed by 2% agarose electrophoresis with Tris-borate buffer at 150 volts for 45 minutes.

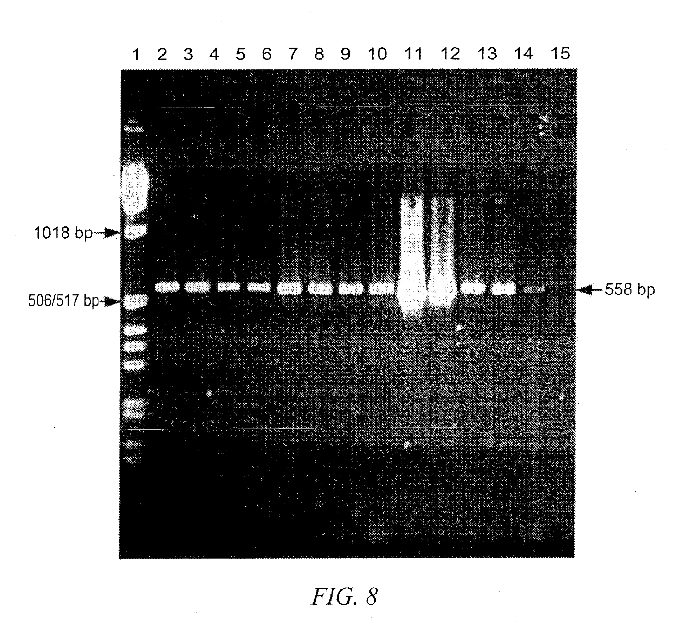

Referring to **Figure 8**, Lane 1 contains a double stranded DNA molecular weight marker. Lanes 2-9 contain PCR products from DNA extracted after 25 days dry storage using the nanoparticles of Example 5. The products in lanes 2-5 are the result of storage at 56°C, and the products in lanes 6-9 are the results of storage at room temperature. Lanes 2, 4, 6, and 8 contain the PCR products from 1 ng of extracted DNA, and lanes 3, 5, 7, and 9 contain the PCR products from 3 ng of extracted DNA. Lanes 10-14 are PCR controls containing known amounts of human DNA with 10 ng in lane 10, 1.0 ng in lane 11, 0.2 ng in lane 12, 0.1 ng in lane 13 and 0.01 ng in lane 14. Lane 15 is a negative PCR control with no DNA added.

As seen in **Figure 8**, these data demonstrate reversible DNA recovery from a ZrO₂ nanoparticle matrix passivated with borax with the addition of glycerol as a plasticizer, as in Example 5, out to 25 days of room temperature dry state storage. The data also show that, with the nanoparticle formulation, use, recovery with the ZrO₂ nanoparticle matrix, with the addition of glycerol, approached 100%. The data show that DNA recovery and the quality of the recovered DNA, as a PCR template, were not affected by raising the storage temperature to 56°C.

### Example 17: DNA Storage on Nanoparticle Plates

DNA is stored by adding to 200 µL or less of purified DNA (up to 1 µg), 125 µg of borate-passivated zirconium, 40 µg of disodium tetraborate (borax), and 75 µg of glycerol as a 20 µL suspension. After adding the mixture to the DNA, allow to air dry, and store at room temperature. To recover the DNA, add 10 µL to 100 µL of water and resuspend the particle to suspension. Pellet the nanoparticle from the suspensions and remove the DNA solution.

### Example 18: RNA Storage on Nanoparticle Plates

Two total RNA samples were used to assess the ability to recover RNA for RT-PCR and real-time quantitative PCR analysis. Sample one was a purchased fetal liver total RNA with no added RNA stabilizing agents. Sample two was pooled total RNA from extracted 50 µL bloodstains obtained from three male newborn (1-day old) individuals, solubilized in an RNA stabilizing solution. 10-, 25-, 50-, and 100-ng of each sample, concentrated in 25 µL nuclease-free water, was added in duplicate to the 96-well plates coated with the dried nanoparticle matrix. The plates were allowed to dry at room temperature in Brittan bags containing desiccant pouches. Duplicate RNA samples were stored at -20°C for RNA stability controls.

After dehydration of the RNA-matrix complex into a 2-mm ceramic disk, the storage plates were sealed with an adhesive cover and stored at either room temperature for ambient storage or at 56°C to simulate "accelerated" extended interval storage conditions. The 56°C plate was used to make stability predictions, since chemical reaction rates (for first order reactions) generally double with each 10°C, Stability predictions were made from the Arrhenius Equation: Predicted Stability = Accelerated Stability x 2DT/10, wherein DT = Δ between normal (22°C) and sample (56°C) storage temperatures.

The RNA was eluted at 1-, 3-, 7-, 14-, 21-, and 28-days by the addition of 20- to 50-µl nuclease-free water and pipette mixing. The matrix was removed from the sample by brief centrifugation, and the supernatant containing the RNA was vacuum centrifuged and resuspended in 10 µl of nuclease-free water.

### Example 19: RT-PCR Analysis of Stored RNA Samples

To determine the stability of the two RNA samples in the nanoparticle storage matrix, RNA was reverse-transcribed into a cDNA template and two duplex amplification reactions were performed. For the gel based RT-PCR duplex assay, PCR using a duplex amplification reaction was used to analyze the stability of a ubiquitously expressed housekeeping gene, GNAS, and a tissue specific gene transcript, the gamma hemoglobin newborn isoform HBG2n3 (Alvarez, M., et al., 2007, Molecular origin and expression of four novel gamma hemoglobin isoforms, submitted for publication). For the quantitative real-time PCR (qPCR) duplex assay, PCR was used to analyze the stability of the ubiquitously expressed housekeeping gene, ribosomal protein S15, and a tissue specific gamma hemoglobin newborn isoform, HBGlnl (Alvarez, M., et al., 2006, The identification of newborns using messenger RNA profiling analysis. Analytical Biochemistry 357: 21-34).

High quality and sufficient quantity of RNA was consistently recovered from both RNA samples at all time points from both storage conditions. Stored RNA (≥ 25 ng) was readily detectable and amplifiable after 28-days of ambient and 56°C storage with both PCR and quantitative real-time PCR reactions. The only samples where amplification was not detected were the 10 ng fetal liver RNA input samples in the RT-PCR based assay with the 56°C storage conditions at days 21 and 28 of storage. The 56°C incubation was equivalent to approximately 42 weeks of room temperature storage.

### Example 20: Borate Treated Kaolin Nanoparticles

Acid washed kaolin nanoparticles were prepared by first suspending the kaolin (CAS# 1332-58-7) nanoparticles, Englehardt, ASP G90 in deionized water at a weight to volume ratio of 1:3. This colloidal suspension was incubated for a minimum of 16 hours. The nanoparticles were then washed by a sedimentation-resuspension process. This process included sedimenting the nanoparticles out of suspension by centrifugation at 4000 G for 10 minutes, resuspending the kaolin in water at the same ratio, and repeating this process until the supernatant were clear with no sign of opalescence. The final kaolin pellet was resuspended at 1 to 3 ratio of weight per volume in water. Then an equal volume of 10% sulfuric acid was added to the suspension. This sulfuric acid/kaolin slurry was mixed and incubated at room temperature from I to 2 hours. Then the slurry was washed with distilled water by the sedimentation-resuspension process until the pH of the supernatant was the same as the pH of the distilled water. To this suspension, 1/50 volume of 500 mM NaF was added at a 1 to 10 ratio. The suspension was mixed and incubated. Then the suspension was subjected to one round of sedimentation-resuspension with distilled water, with the pellet being resuspended in 100 mM borate buffer (1:1 mixture of 100 mM boric acid to 100 mM sodium tetraborate) at ratio of I to 10 and mixed for at least 16 hours. This suspension was subjected to three rounds of sedimentation-resuspension with 10 mM borate buffer. The particles were stored in this condition until ready for dilution in 10 mM borate buffer.

### Example 21: Storage of Total Serum Proteins on Nanoparticle Plates

For 50 µL of fluid serum (5 mg of total protein), 50 µL of a suspension consisting of 1 mg of borate-passivated kaolin (Example 20), 2 mg of borax, and 3.5 mg of glycerol is added, and the mixture is allowed to air dry. To recover the serum proteins, water is added to at least 20 µL, and the particles are gently agitated back into colloidal suspension.

### Example 22: Storage of Serum Antibody on Nanoparticle Plates

For 100 µL of serum, add 50µL of 2 M sodium sulfate, up to 20 mg of kaolin nanoparticles (200 nm diameter) coated with mercaptosilane, further functionalized in sequential order with divinyl sulfone, followed by mercaptoethanol. After the serum proteins adsorb to the surface ligands, these ligand bound proteins are concentrated and enriched, first by sedimentation of particles from suspension. Then these particles are washed in 100 mM NaCl three times by sedimentation-resuspension and are added to the 100 µL of serum diluted in PBS to 800 mM in which 200 µL of 2 M sodium sulfate solution is added. The thiophilic ligand coated kaolin particles are added as a suspension in PBS with 0.5 M sodium sulfate in a volume not to exceed 500 µL. After at least a 30 minute incubation, the particles are sedimented at 4000 G for 5 minutes, resuspended in PBS and 0.5 M sodium sulfate and sedimented at 4000 G. The resulting pellet is resuspended in 100 µL of a solution containing 2 mg of thiophilic kaolin, 2 mg of borax, and 3.5 mg of glycerol. The suspension is allowed to dry at room temperature to dryness. The immunoglobulins are recovered by resuspending the pellet in at least 50 µL of 100 mM NaCl.

### Example 23: Dry State Storage of Cell or Tissue Lysates with Kaolin Nanoparticles in a 96 Well Format for Release and Purification at a Later Date

Blood cells, whole blood, frozen blood, or cheek cell samples collected by either mouth wash rinse or swabs, and are lysed by resuspension in a solution containing 20 mM CAPS, 20 mM Na₂CO₃, 20 mM EDTA, 2% sodium lauroyl sarcosyl, and 1.8 M guanidinium hydrochloride. The resulting solution of lysed cells or tissue is then diluted 1:1 with water. 20% by volume of Savinase protease solution is then added. Savinase is a bacterial protease from *Baccillus* species used at 16U/gm. Between I mg to 5 mg of borate-passivated kaolin is added to this cell lysate and protease solution. The slurry is then aliquoted into one well of a 96 well microtiter plate at a volume not to exceed about 200 µL. The plate is then allowed to dry at room temperature. Alternatively, the plate is placed at 56°C until dry.

The resulting dry lysate is recovered from a well by the addition of a volume of water equal to the original fluid volume prior to drying (up to 200 µL). The cell lysate is then processed for DNA isolation by dilution to 500 µL with the addition of 10 mM CAPS, 10 mM Na₂CO₃, 10 mM EDTA, 1% sodium lauroyl sarcosyl, 0.9M guanidinium hydrochloride, and 50 µL of Savinase. The diluted sample is then incubated at room temperature for 30 minutes with periodic mixing until a homogeneous suspension is formed. The suspension is then transferred to a new vessel, then further incubated for one hour up to 16 hours at 56°C. Kaolin particles are removed from the Savinase digestion product supernatant by centrifugation for 5 minutes at 5,000 G. The resulting DNA-containing supernatant is then transferred to standard microfuge tube. To this supernatant is added up to I mg of phosphate-passivated kaolin nanoparticles. LiCl (from a 10 M solution) is added to a final concentration of 0.5 M, followed by the addition of 1 volume of isopropanol. This is mixed to form a suspension. The suspension is incubated for 30 minutes at room temperature, and then centrifuged at 4,000 G for 5 minutes to form a nanoparticle pellet at the bottom of the tube. The DNA-containing nanoparticle pellet is retained. The pellet is then treated with a solution of 50% ethanol/0.15 M NaCl and then centrifuged at 4,000 G for 2 minutes. The pellet is retained and air dried for ten minutes. To the air-dried pellet is added at least 20 µL up to about 200 µL of an elution buffer comprising 10 mM Na Borate at pH 9 and 0.1 mM EDTA. The particles are resuspended in this buffer for 15 to 30 minutes at 56°C and mixed until a colloidal suspension is reformed. The particles are sedimented by centrifugation at 4,000 G for 5 minutes, and the DNA containing supernatant is harvested by pipetting. DNA in that final eluate is then ready for use for applied genetic analysis or preparative DNA biochemistry.

All patents and publications, including all sequences disclosed within such patents and publications, referred to herein are expressly incorporated by reference.

Although the invention has been described with reference to the presently preferred embodiments and the foregoing non-limiting examples, it should be understood that various changes and modifications, as would be obvious to one skilled in the art, can be made without departing from the spirit of the invention. Accordingly, the invention is limited only by the following claims.

The invention furthermore comprises the following items:
**1.** A composition useful for biomolecule storage comprising a population of nanoparticles, wherein the surface of the nanoparticles are passivated so that they are inert to a biomolecule.
**2.** The composition of item **1,** wherein the nanoparticles have an average diameter of about 25 nm.
**3.** The composition of item **1,** wherein the nanoparticles have a diameter range from about 20 nm to about 100 nm.
**4.** The composition of item **1**, wherein the nanoparticles comprise ceramic material.
**5.** The composition of item **1,** wherein the nanoparticles comprise aluminosilicate or metal oxide.
**6.** The composition of item **1,** wherein the nanoparticles comprise a material selected from the group consisting of tungsten oxide, zirconium oxide, aluminum oxide, titanium oxide, tin oxide, phylosilicate, smectite, kaolin, bentonite, and combinations thereof.
**7.** The composition of item **1,** wherein the nanoparticles comprise branched polymers of a sugar, dextran or cellulose.
**8.** The composition of item **1,** wherein the nanoparticles are passivated by an oxyanion.
**9.** The composition of item **1,** wherein the nanoparticles are passivated by borate, phosphate, sulfate, citrate, or fluoride.
**10.** The composition of item **1,** wherein the nanoparticles are passivated by an inactivating agent.
**11.** The composition of item **1,** wherein the composition comprises an inactivating agent and wherein the surface of the nanoparticles are passivated by the inactivating agent.
**12.** The composition of item **1,** wherein the composition is an aqueous suspension.
**13.** The composition of item **1,** wherein the composition is a dry storage matrix.
**14.** The composition of item **1,** wherein it is provided in a multi-compartment container.
**15.** The composition of item **1,** wherein the biomolecule is a polynucleotide or polypeptide.
**16.** The composition of item **1** further comprises a biomolecule.
**17.** The composition of item **1** further comprises a biomolecule in the presence of an inactivating agent, wherein the surface of the nanoparticles are passivated by the inactivating agent.
**18.** The composition of item **1** further comprises a small molecule stabilizer.
**19.** The composition of item **1** further comprises a small molecule stabilizer and a small molecule additive.
**20.** The composition of item **1** further comprises a biomolecule and a small molecule stabilizer.
**21.** The composition of item **1** further comprises a biomolecule and a small molecule stabilizer selected from the group consisting of boric acid, sodium borate, boric acid-glycerol, boric acid-1,3 propanediol, sodium phosphate, CAPS, Na₂CO₃, EDTA, sodium lauroyl sarcosyl, guanidinium hydrochloride, SDS, urea, Tris, and combinations thereof.
**22.** The composition of item **1** further comprises a biomolecule, a small molecule stabilizer, and a small molecule additive.
**23.** The composition of item **1** further comprises a biomolecule, a small molecule stabilizer, and a small molecule additive selected from the group consisting of polyols, simple monosaccharides, disaccharides, complex sugars and dextrans.
**24.** The composition of item **16,** wherein the biomolecule is a polynucleotide or a polypeptide.
**25.** The composition of item **16,** wherein the composition is a dry storage matrix.
**26.** The composition of item **16,** wherein the biomolecule is a polypeptide and the nanoparticles are branched polymers of a sugar, dextran, or cellulose.
**27.** The composition of item **26,** wherein the nanoparticles are polysucrose, Ficoll, or agarose.
**28.** The composition of item **1** further comprises a biomolecule in a biological lysate.
**29.** A method of storing a biomolecule comprising:
   mixing a composition comprising a biomolecule with the composition of item **1** to form a mixture; and
   drying the mixture for storage.
**30.** The method of item **29,** wherein the composition comprising a biomolecule is mixed with a small molecule stabilizer.
**31.** The method of item **29,** wherein the composition comprising a biomolecule is a biological lysate.
**32.** The method of item **29,** wherein the composition comprising a biomolecule is a biological lysate comprising an inactivating agent.
**33.** A multi-compartment container comprising a dry storage matrix in a compartment of the container, wherein the dry storage matrix comprises the composition of item **1**.
**34.** A multi-compartinent container comprising a dry storage matrix in a compartment of the container, wherein the dry storage matrix comprises the composition of item **16**.

## Claims

1. A method for storing biomolecules from a biological sample, comprising the steps of:
a) providing a population of nanoparticles wherein the surface of the nanoparticles are passivated so that they are inert to a biomolecule;
b) treating the biological sample with the surface-passivated nanoparticles;
c) drying the biological sample containing the surface-passivated nanoparticles;
d) storing the dried biological sample containing the surface-passivated nanoparticles.

2. The method of claim 1 further comprising the steps of:
e) hydrating the stored, dried biological sample containing the surface-passivated nanoparticles;
f) eluting the biomolecules from the surface-passivated nanoparticles; and
g) recovering the eluted biomolecules.

3. The method of claim 1 or claim 2 wherein the nanoparticles have an average diameter of about 25 nm.

4. The method of claim 1 or claim 2 wherein the nanoparticles a diameter range from about 20 nm to about 100 nm.

5. The method of any one of claims 1 to 4, wherein the nanoparticles comprise ceramic material.

6. The method of any one of claims 1 to 5, wherein the nanoparticles comprise aluminosilicate or metal oxide.

7. The method of any one of claims 1 to 4, wherein the nanoparticles comprise a material selected from the group consisting of tungsten oxide, zirconium oxide, aluminum oxide, titanium oxide, tin oxide, phylosilicate, smectite, kaolin, bentonite, and combinations thereof.

8. The method of any one of claims 1 to 4, wherein the nanoparticles comprise branched polymers of a sugar, dextran or cellulose.

9. The method of any one of claims 1 to 8, wherein the nanoparticles are passivated with an oxyanion.

10. The method of claim 9, wherein the nanoparticles are passivated with borate, phosphate, sulfate, citrate, fluoride, or combinations thereof.

11. The method of any one of claims 1 to 8, wherein the nanoparticles are passivated with an inactivating agent.

12. The method of claim 8, wherein the biomolecule is a polynucleotide or polypeptide.

13. The method of claim 10, wherein the biomolecule is a nucleic acid.

14. The method of claim 9, further comprising the step of:
b1) adding a small molecule stabilizer to the biological sample with the surface-passivated nanoparticles.

15. The method of claim 14, further comprising the step of:
b2) adding a small molecule additive to the biological sample with the surface-passivated nanoparticles.

16. The method of claim 14, wherein the small molecule stabilizer is selected from the group consisting of boric acid, sodium borate, boric acid-glycerol, boric acid-I ,3 propanediol, sodium phosphate, CAPS, Na2C03, EDTA, sodium lauroyl sarcosyl, guanidinium hydrochloride, SDS, urea, Tris, and combinations thereof.

17. The method of claim 15, wherein the small molecule additive is selected from the group consisting of polyols, simple monosaccharides, disaccharides, complex sugars, and dextrans.

18. The method of claim 17, wherein the small molecule additive is glycerol.

19. The method of claim 8, wherein the nanoparticles further comprise polysucrose, Ficoll, agarose, or combinations thereof.

20. The method of any one of claims 1 to 19, wherein the biological sample is selected from the group consisting of blood, plasma, urine, saliva, spinal fluid, a biological fluid, skin cells, a cell sample, a tissue sample, a cell lysate, a nuclear extract, a nucleic acid extract, a protein extract, a cytoplasmic extract, or combinations thereof.
